(19) Europäisches Patentamt
European Patent Office
Office européen des brevets

(11) EP 0 893 983 B1

(12) **EUROPEAN PATENT SPECIFICATION**

(45) Date of publication and mention of the grant of the patent:
**05.12.2001 Bulletin 2001/49**

(21) Application number: **97914603.2**

(22) Date of filing: **04.04.1997**

(51) Int Cl.7: **A61K 7/00**, A61K 7/06

(86) International application number:
**PCT/JP97/01164**

(87) International publication number:
**WO 97/38667 (23.10.1997 Gazette 1997/45)**

(54) **HAIR COSMETIC COMPOSITION**

HAARKOSMETISCHE ZUSAMMENSETZUNG

COMPOSITION DE SOIN CAPILLAIRE

(84) Designated Contracting States:
**DE ES FR GB**

(30) Priority: **18.04.1996 JP 9710096**
**23.08.1996 JP 22276996**
**03.09.1996 JP 23298196**

(43) Date of publication of application:
**03.02.1999 Bulletin 1999/05**

(73) Proprietor: **Kao Corporation**
**Chuo-Ku Tokyo 103 (JP)**

(72) Inventor: **TAKAHASHI, Toshie,**
**c/o Kao Corporation**
**Tokyo 131 (JP)**

(74) Representative:
**Kindler, Matthias, Dr. Dipl.-Chem. et al**
**Hoffmann Eitle,**
**Patent- und Rechtsanwälte,**
**Arabellastrasse 4**
**81925 München (DE)**

(56) References cited:

| | |
|---|---|
| **EP-A- 0 288 012** | **EP-A- 0 432 951** |
| **EP-A- 0 468 721** | **EP-A- 0 573 229** |
| **US-A- 4 983 377** | **US-A- 5 441 728** |

- **PATENT ABSTRACTS OF JAPAN vol. 018, no. 118, 25 February 1994 & JP 05 310533 A (TOSHIBA SILICONE), 22 November 1993, cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 893 983 B1

## Description

TECHNICAL FIELD

The present invention relates to a hair treatment composition which provides superior natural gloss or luster to hair and enhanced hair-dressing ability, and can prevent hair damage and repair damaged hair.

BACKGROUND ART

Hair cosmetic compositions are required to have such characteristics that they impart good gloss or luster to hair and have enhanced hair-dressing ability. In order to give hair gloss, it has heretofore been proposed to use a hair styling composition containing an oily substance or a polymer to coat the hair, thereby enhancing the reflection of light. For example, EP-A-688557 describes an aqueous hair spray composition comprising (I) a water-soluble polymer having a solution viscosity at 10% in water of less than about 20,000 cps at 25°C, the polymer being present in an effective amount for setting hair; and (ii) a latex of water-insoluble polymeric particles dispersed in water, the average particle size being no higher than about 3 microns, the particles having a glass transition temperature of 250 to 300°K and being present in an effective amount to interact with the polymer to further improve the setting of hair. Japanese Patent Application Laid-Open No. 184115/1984 describes a hair cosmetic composition comprising 0.1-20·wt.% of spherical particles of a water-insoluble polymer and 0.1-15 wt.% of a cationic compound. Japanese Patent Application Laid-Open No. 310533/1993 describes a hair cosmetic composition comprising a powder of hardened polyorganosiloxane.

According to these methods, however, the intensity of reflected light at completely coated portions of the hair becomes several times as high as that of the original hair, showing an unnatural look. In some cases, the composition adheres incompletely and heavily to the hair, resulting in a low reflection of light. In any event, the luster of the coated hair differs from the original hair in quality and feeling, and the appearance becomes unnatural. Additionally, due to the film of the oily substance or polymer, hairs stick to each other giving dirty appearance.

On the other hand, in order to enhance hairdressing ability, it has been proposed to incorporate an oily substance or silicone into a shampoo, hair rinse, hair treatment composition or hair styling composition to cause such a component to be adsorbed on hair. However, this method has also involved a problem that the composition gives users an unpleasant feel such as a sticky feel of the whole hair.

Accordingly, it is an object of the present invention to provide a hair cosmetic composition which imparts excellent natural gloss or luster to hair and enhanced hair-dressing ability.

In view of the foregoing circumstances, the inventor has carried out an extensive investigation with a view toward solving the above problems.

Each hair fiber is coated with a layer of flaky cells called cuticle overlapping one another in the form of a plate. Human hair is coated with generally 5-10 layers of cuticles. A part of the outermost layer is exposed to the outside. Of the exposed part, the outer periphery of the cuticle cell is at an angle almost perpendicular to the axis of the hair. This part will hereinafter be referred to as an edge part. Portions other than the outer periphery are at a gentle angle of about 5° with respect to the axis of the hair. These portions will hereinafter be referred to as a flat part of the cuticle. This flat part of the cuticle occupies most of the exposed area.

The inventor has thus found that when hair is treated with a composition containing fine particles of a water-insoluble polymer or inorganic material having a particle diameter substantially equal to the height (0.2-1 μm) of the edge part, the fine particles are scarcely adsorbed on the flat part of cuticle and selectively adsorbed on the edge part. It has also been found that when the fine particles are selectively adsorbed on the edge part, the steep angle of the edge part is moderated to improve the smoothness of hair fibers, thereby imparting good gloss or luster to the hair. It has been further found that when the fine particles are caused to be adsorbed only on the edge part while leaving the flat part of cuticle, which occupies the most area of the hair surface, intact without coating the whole hair with the oily substance or polymer like the conventional hair styling compositions, thereby moderating the angle of the edge part, natural gloss or luster can be imparted to the hair without strengthening the intensity of reflected light to an unnatural level when exposing the hair to light.

According to the present invention, there is thus provided a hair cosmetic composition comprising fine particles of a water-insoluble polymer or inorganic material, which have an average particle diameter not smaller than 0.2 μm, but smaller than 1 μm.

According to the present invention, there is also provided a method of imparting gloss or luster to hair, which comprises applying fine particles of a water-insoluble polymer or inorganic material, which have an average particle diameter not smaller than 0.2 μm, but smaller than 1 μm, to the hair.

BRIEF DESCRIPTION OF DRAWINGS

FIG. 1 is an electron micrograph illustrating Invention Product 9 as adsorbed on hair.
FIG. 2 is an electron micrograph illustrating Invention Product 10 as adsorbed on hair.
FIG. 3 is an electron micrograph illustrating Comparative Product 6 as adsorbed on hair.
FIG. 4 is an electron micrograph illustrating Comparative Product 7 as adsorbed on hair.

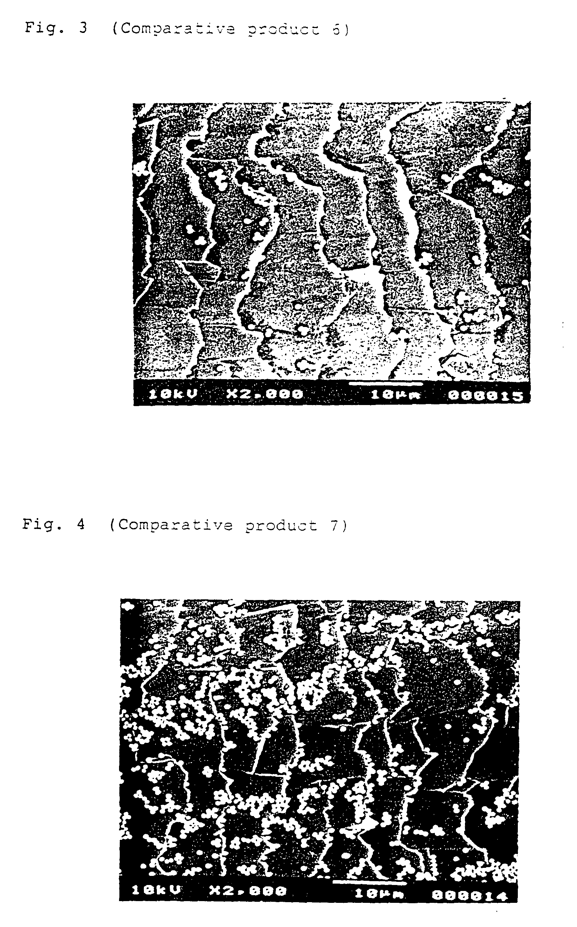

The fine particles useful in the practice of the present invention are required to have an average particle diameter not smaller than 0.2 μm, but smaller than 1 μm, preferably 0.3-0.9 μm. If the average particle diameter is smaller than 0.2 μm, the effects of imparting gloss or luster and improving the smoothness of hair cannot be sufficiently brought about. If the average particle diameter is not smaller than 1 μm on the other hand, the number of fine particles adsorbed on the hair decreases, so that the smoothness of the hair cannot be improved, resulting in a hair cosmetic composition failing to impart satisfactory gloss or luster. With respect to the particle size distribution of these fine particles, it is preferable from the viewpoint of the effect of imparting good gloss or luster that at least 80% of the fine particles be within a range of the average particle diameter ± 30%.
The average particle diameter and particle size distribution can be determined by means of a scanning electron microscope in the following manner.
A suspension of fine particles to be measured is applied on sample carriers for the scanning electron microscope to prepare such samples that the microscope can count several tens of particles in a visual field of 10,000 magnifications. Ten micrographs of the sample are taken in different visual fields to determine a root mean square particle diameter $\bar{d}$ defined by the following equation by means of an image analyzer.

$$\bar{d} = \frac{\sum n_i d_i^2}{\sum_i n_i d_i}$$

wherein $n_i$ is the number of particles having a particle diameter of $d_i$.
No particular limitation is imposed on the form or shape of the fine particles; they may be in any form, e.g., spherical, amorphous, flat and plate forms, etc. They may also have any structure such as hollow and porous structures and the like. The fine particles may be subjected to surface modification, a chemical treatment for-imparting porosity, oil absorbing ability or water absorbing ability before use.
Among the fine particles used in the present invention, examples of the fine particles of the water-insoluble polymer include fine particles of polymers such as silicone resins, polymers of alkyl acrylates and copolymers with polymerizable monomers copolymerizable therewith, polymers of alkyl methacrylates and copolymers with polymerizable monomers copolymerizable therewith, polymers of styrene and copolymers with polymerizable monomers copolymerizable therewith, and chitosanmethacrylic acid copolymers.
Of these, as the silicone resins, there may be used water-insoluble silicone resins having a structure three-dimensionally crosslinked by siloxane bonds. Commercially-available products, for example, Tospearl® produced by Toshiba Silicone Co., Ltd., KMP590 produced by Shin-Etsu Chemical Co., Ltd., and Trefil® R produced by Dow Corning Toray Silicone Co., Ltd., may be used.
The alkyl methacrylates with which respective alkyl methacrylate polymers are formed include methyl methacrylate, ethyl methacrylate, propyl methacrylate, n-butyl methacrylate, n-hexyl methacrylate, n-octyl methacrylate, 2-ethylhexyl methacrylate and n-lauryl methacrylate. Similarly, the alkyl acrylates with which respective alkyl acrylate polymers are formed include methyl acrylate, ethyl acrylate, propyl acrylate, n-butyl acrylate, n-hexyl acrylate, n-octyl acrylate, 2-ethylhexyl acrylate and n-lauryl acrylate.
Preferable alkyl methacrylate polymers are polymethyl methacrylate and polyethyl methacrylate, with polymethyl methacrylate being particularly preferred. Preferable alkyl acrylate polymers are polymethyl acrylate and polyethyl acrylate.
An example of a monomer copolymerizable with the alkyl acrylate or alkyl methacrylate is styrene. Exemplary preferable copolymers thereof include styrene-butyl methacrylate copolymers. Examples of the monomers copolymerizable with styrene include divinylbenzene and the like. Preferable copolymers of styrene include styrene-divinylbenzene copolymers.
Examples of the fine particles of the inorganic material include fine particles of titanium oxide, silica, alumina,

iron oxide, zinc oxide, chromium oxide and mica.

The fine particles used in the present invention may or may not be charged with positive or negative electricity, like the uncharged fine particle as described above, but may be charged with positive or negative electricity. An example of the fine particles negatively charged, i.e., anionic fine particles, includes MP-1000 produced by Soken Chemical & Engineering Co., Ltd., which are fine particles of polymethyl methacrylate negatively charged. Fine particles of water-insoluble cationic polymers include fine particles of polymers having a cationic group, such as a primary, secondary or tertiary amino group, or quaternary ammonium group, on at least part of their main chains or side chains. Specific examples thereof include amine-added acrylic resins.

In the present invention, there may be further used fine particles of a water-insoluble polymer or inorganic material, which have been subjected to a surface treatment with a cationic, anionic and/or amphoteric compound.

Examples of the cationic compound used in the surface treatment herein include cationic surfactants and cationic polymers.

The cationic surfactants include, for example, quaternary ammonium salts represented by the following general formulae (1) and (2):

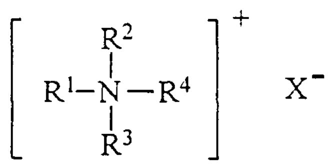
(1)

wherein at least one of $R^1$, $R^2$, $R^3$ and $R^4$ is an alkyl or alkenyl group which has 8-28 carbon atoms in total and may be substituted by an alkoxy, alkenyloxy, alkanoylamino or alkenoylamino group, the remainder are independently a benzyl group or an alkyl or hydroxyalkyl group having 1-5 carbon atoms, and $X^-$ is a halide ion or organic anion.

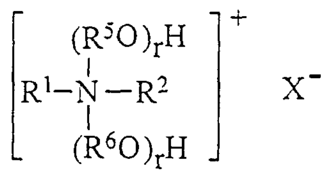
(2)

wherein at least one of $R^1$ and $R^2$ is an alkyl or alkenyl group which has 8-28 carbon atoms in total and may be substituted by an alkoxy, alkenyloxy, alkanoylamino or alkenoylamino group, the remainder is a benzyl group or an alkyl or hydroxyalkyl group having 1-5 carbon atoms, $R^5$ and $R^6$ are independently an alkylene group having 2-3 carbon atoms, X- is a halide ion or organic anion, and r, is an integer of 1-20.

Specific examples of $X^-$, which is a counter ion to each of the quaternary ammonium salts represented by the general formulae (1) and (2), include halide ions such as chloride, iodide and bromide ions; and organic anions such as methosulfates, ethosulfates, methophosphates and ethophosphates.

The cationic polymers include, for example, canonized cellulose derivatives, canonized starch, cationized guar gum derivatives, diallyl quaternary ammonium salt/acrylamide copolymers, quaternized poly(vinyl pyrrolidone) derivatives, polyglycol-polyamine condensates.

Specific examples thereof include poly(dimethyldiallylammonium halide) type cationic polymers represented by the following general formula (3) or (4):

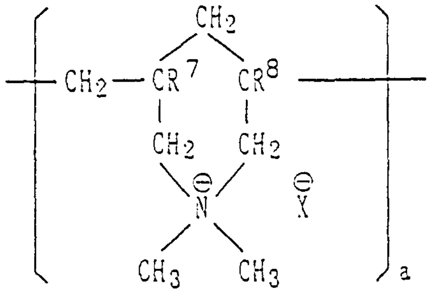

(3)

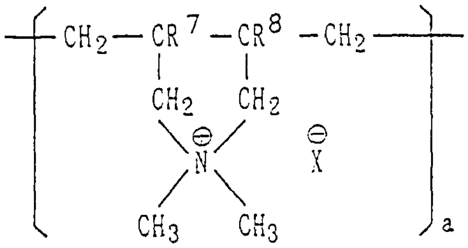

(4)

wherein $R^7$ and $R^8$ are independently a hydrogen atom or a methyl group, X is a halogen atom, and a is a number of 150-6,200; dimethyldiallylammonium halide-acrylamide copolymer type cationic polymers represented by the following general formula (5) or (6) :

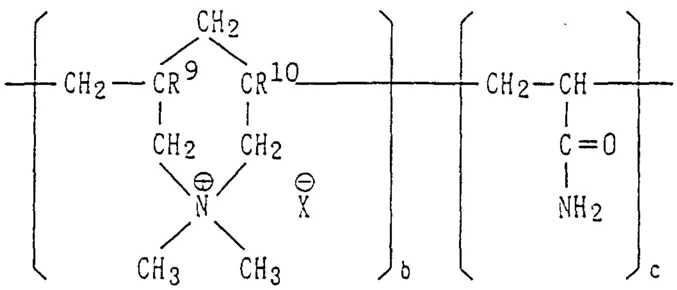

(5)

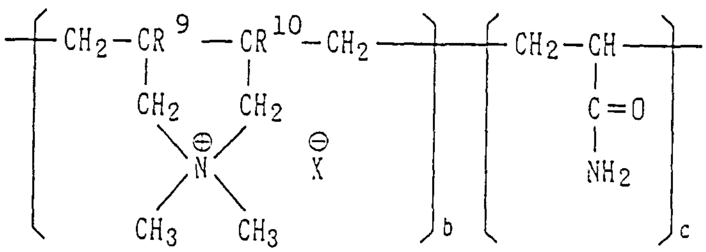

(6)

wherein $R^9$ and $R^{10}$ are independently a hydrogen atom or a methyl group, X is a halogen atom or an organic anion, b + c is a number of 150-9,000; quaternary nitrogen-containing cellulose ethers; condensation products of tallowylamine obtained from polyethylene glycol, epichlorohydrin, propyleneamine and beef tallow fatty acid; condensation products of cocoylamine obtained from polyethylene glycol, epichlorohydrin, propyleneamine and coconut oil fatty acid; and canonized products of vinylpyrrolidone-dimethylaminoethyl methacrylate copolymers.

Examples of the anionic compound include anionic surfactants and anionic polymers commonly used in classical hair cosmetic compositions, and the like. No particular limitation is imposed on the anionic surfactants used herein so far as they are those commonly used in the classical shampoo compositions and the like. Examples thereof include the following anionic surfactants (i) to (xi):

(i) linear or branched alkylbenzenesulfonates having an alkyl group having 10-16 carbon atoms on the average;

(ii) alkyl or alkenyl ether sulfates having an alkyl or alkenyl group having 10-20 carbon atoms on the average, to which ethylene oxide, propylene oxide, butylene oxide, or ethylene oxide and propylene oxide at a ratio of 0.1/9.9 to 9.9/0.1 or ethylene oxide and butylene oxide at a ratio of 0.1/9.9 to 9.9/0.1 are added in a proportion of 0.5-8 mol on the average per molecule;
(iii) alkyl- or alkenylsulfates having an alkyl or alkenyl group having 10-20 carbon atoms on the average;
(iv) olefinsulfonates having 10-20 carbon atoms on the average per molecule;
(v) alkanesulfonates having 10-20 carbon atoms on the average per molecule;
(vi) saturated or unsaturated fatty acid salts having 10-24 carbon atoms on the average per molecule;
(vii) amidoether carboxylic acid type surfactants represented by the following formula (7):

$$R^{11}\text{-}(OC_3H6)d\text{-}(OC_2H_4)_e\text{-}OCH_2\text{-}COOA \qquad (7)$$

wherein $R^{11}$ is a linear or branched alkyl or alkenyl group having 8-22 carbon atoms, $C_{8-9}$-alkylphenyl or $R^{12}CONH\text{-}CH_2\text{-}CH_2\text{-}$ in which $R^{12}$ is a linear or branched alkyl or alkenyl group having 11-21 carbon atoms, d is a number of 2-24, e is a number of 0-6, and A is a hydrogen atom, sodium, potassium, lithium, magnesium, a residue of monoethanolamine, ammonium or a residue of triethanolamine;
(viii) $\alpha$-sulfo-fatty acid salts or esters having an alkyl or $\alpha$-alkenyl group having 10-20 carbon atoms on the average;
(ix) N-acylamino acid type surfactants having an acyl group having 8-24 carbon atoms and a free carboxylic acid residue;
(x) mono- or diphosphate type surfactants having an alkyl or alkenyl group having 8-24 carbon atoms; and
(xi) the sulfosuccinates of higher alcohols having 8-22 carbon atoms, ethoxylates thereof or the like, or sulfosuccinates derived from higher fatty acid amides.

Examples of counter ions to the anionic residues of these anionic surfactants include ions of alkali metals such as sodium and potassium, ions of alkaline earth metals such as calcium and magnesium, an ammonium ion, and alkanolamines (for example, monoethanolamine, diethanolamine, triethanolamine, triisopropanolamine, etc.) having 1-3 alkanol groups having 2 or 3 carbon atoms.

As the anionic polymers, alkyl vinyl ether-maleic acid copolymers are preferred, with a methyl vinyl ethermaleic acid copolymer being particularly preferred. This copolymer is obtained by the hydrolysis of a copolymer of vinyl ether with maleic anhydride and is commercially available under the trade names of Gantrez® AN or ES. These copolymers may be partially esterified, for example, by ethyl, butyl or isobutyl moiety (for example, Gantrez® ES225). Further examples of the anionic polymers include vinyl acetate-crotonic acid copolymers and vinyl acetate-vinyl neododecanoatecrotonic terpolymers (known as Resins® series); sodium acrylate-vinyl alcohol copolymers (known as Hydagen® F); sodium polystyrenesulfonate (known as Flexane® 130); ethyl acrylate-acrylic acid-N-tert-butylacrylamide terpolymers (known as Ultrahold®); vinylpyrrolidone-vinyl acetate-itaconic acid terpolymers; and acrylic acidacrylamide copolymers and their sodium salts (known as Reten®).

Examples of the amphoteric compound include amphoteric surfactants and amphoteric polymers commonly used in the classical hair cosmetic compositions. The amphoteric surfactants include carbobetaine type surfactants and sulfobetaine type surfactants. The amphoteric polymers include, for example, N-octylacrylamide-(meth)acrylic acid-tert-butylaminoethyl methacrylate terpolymers (known as Amphomer®); methacryloyloxyethylbetaine-alkyl methacrylate copolymer (known as Yukaformer®), more specifically methacryloyloxyethylbetaine-butyl methacrylate copolymers (known as Yukaformer® Am75); copolymers of monomers having a carboxylic acid or sulfonic acid, more-specifically copolymers of (meth)acrylic acid and/or itaconic acid with an amino group-containing monomer such as a mono- or dialkylaminoalkyl (meth)acrylate or dialkylaminoalkyl(meth)acrylamide; copolymers of N-octylacrylamide, methyl methacrylate, hydroxypropyl methacrylate, N-tert-butylaminoethyl methacrylate and acrylic acid; and copolymers described in U.S. Patent No. 3,927,199.

The surface treatment with any of these cationic, anionic or amphoteric compounds may preferably be performed by using the fine particles and the ionic compound at a weight ratio of 1/20 to 20/1.

The hair cosmetic composition according to the present invention may further contain a cationic, anionic or amphoteric compound in addition to the fine particles. Examples of such cationic, anionic and amphoteric compounds include the same compounds as those used in the surface treatment of the fine particles.

Preferable fine particles useful in the practice of the present invention include fine particles of a water-insoluble polymer selected from the group consisting of the above-mentioned silicone resins, polymers of alkyl acrylates and copolymers with polymerizable monomers copolymerizable therewith, polymers of alkyl methacrylates and copolymers with polymerizable monomers copolymerizable therewith, polymers of styrene and copolymers with polymerizable monomers copolymerizable therewith, and chitosan-methacrylic acid copolymers; and fine particles of an inorganic material

selected from the group consisting of the above-mentioned titanium oxide, silica, alumina, iron oxide, zinc oxide, chromium oxide and mica.

Taking into consideration the adsorption efficiency of the fine particles on hair and preferable effects of avoiding a "squeaky" feel and tight or stiff feel of the hair after application to the hair, more preferable fine particles used in the present invention include fine particles of the cationic polymers; and fine particles obtained by subjecting the fine particles of the water-insoluble polymers or inorganic materials as mentioned above to a surface treatment with a cationic compound. Besides, incorporation of cationic compounds into the hair cosmetic composition in addition to any of the fine particles of the water-insoluble polymers or inorganic materials as mentioned above can bring about the improvement of said properties.

No particular limitation is imposed on the amount of the fine particles to be incorporated into the hair cosmetic composition according to the present invention. However, it is preferable to use the fine particles in an amount of 0.001-10 wt.% from the viewpoints of the effect of imparting gloss or luster to hair and the inhibitory effects on the squeaky feel and tight or stiff feel. It is more preferable to vary the amount of the fine particles according to the usage of the hair cosmetic composition. In the case of a hair cosmetic composition of the type that is rinsed out with water after being applied to hair, such as a hair rinse, a hair conditioner or a rinse-in-shampoo, the fine particles are preferably incorporated in an amount of 0.01-10 wt.%. On the other hand, in the case of a hair cosmetic composition of the type that it is not rinsed out after being applied to hair, such as a hair treatment composition or hair spray composition, the fine particles are preferably incorporated in an amount of 0.001-0.1 wt.%.

When the cationic compound, anionic compound and/or amphoteric compound are incorporated in addition to the fine particles, these ionic compounds are preferably incorporated in an amount of 0.001-20 wt.%.

In the hair cosmetic composition according to the present invention, other ingredients commonly used in the classical hair cosmetic compositions, for example, oily components such as hydrocarbons, higher alcohols, esters and silicone oil; humectants such as glycerol, propylene glycol, 1,3-butylene glycol and polyethylene glycol; surfactants such as anionic surfactants, nonionic surfactants and amphoteric surfactants; medicinally-effective agents such as antidandruff agents and vitamins; antiseptics such as parabens; thickeners such as water-soluble polymers; colorants such as dyes and pigments; pearl-like-hue-imparting agents such as glycol esters; and additionally, chelating agents, various perfume bases and the like, may be suitably incorporated in addition to the above-described components so far as no detrimental influence is thereby imposed on the effects of the present invention.

According to the hair cosmetic composition of the present invention, the water-insoluble fine particles incorporated therein can selectively adsorb on the edge part of hair cuticle, thereby imparting natural gloss or luster to hair, giving users a pleasant feel without any sticky feel and enhancing hair-dressing ability. It is also possible to cause an oily substance to be absorbed on the fine particles in advance so as to cause the oily substance to be adsorbed only on the edge part of cuticle, which tends to be damaged, thereby efficiently exhibiting its repairing effect on the damaged hair.

The words "selective adsorption on the edge part" as used herein means that the amount of the fine particles adsorbed on the edge part amounts to at least 70% of the amount of the fine particles adsorbed on the whole hair cuticle. It is particularly preferable for the amount of the selective adsorption to account for at least 80%. This amount of adsorption can be determined by observing the cuticle, on which the fine particles have been adsorbed, through an electron microscope to count the numbers of fine particles adsorbed on the edge part and flat part of cuticle.

According to the present invention, natural gloss or luster can be imparted to hair by causing the fine particles to be adsorbed on the edge part of hair cuticle. It is however preferable that the fine particles be adsorbed on the edge part in a proportion of 5 particles or more, preferably 15 particles or more per unit surface area, 1,000 $\mu m^2$, of the hair, and at least 70%, preferably at least 80% of the fine particles adsorbed be adsorbed on the edge part. The amount of the fine particles adsorbed per unit surface area of the hair can also be counted by observation through an electron microscope.

## EXAMPLES

The present invention will hereinafter be described in more detail by the following Examples. However, the present invention is not limited to these Examples.

Example 1:

Hair rinse compositions having their corresponding formulations shown in Tables 1 to 4 were prepared in accordance with a method known *per se* in the art to evaluate them as to selective adsorptivity on the edge part, gloss or luster enhancing effect and hair-dressing ability. The results are shown in Tables 1 to 4.

The hair of a monitor was divided into halves, and each of the fine particle-containing hair rinse compositions according to the present invention was applied to one half, while a fine particle-free hair rinse composition (Comparative

Product 1) was applied to the other half. The thus-treated hair was rinsed and dried.

(1) Evaluation of selective adsorptivity on the edge part:

Twenty hairs were collected from the hair portion of the monitor treated with the fine particle-containing rinse composition, and 10 micrographs per hair were taken at 10,000 magnifications where the rinse composition contained fine particles having an average particle diameter smaller than 0.2 μm, at 2,000 magnifications, where the rinse composition contained fine particles having an average particle diameter not smaller than 0.2, but smaller than 1 μm, and at 800 magnification where the rinse composition contained fine particles having an average particle diameter not smaller than 1 μm.

The numbers of particles adsorbed on the edge part and on the whole hair cuticle were counted to find the percentage of the particles adsorbed on the edge part as, an average value on the hair surface in accordance with the following equation, thereby ranking the selective adsorptivity on the edge part in accordance with the following standard.

$$\text{Percentage (\%) of particles adsorbed on the edge part} = \frac{\text{(Number of particles adsorbed on the edge part)}}{\text{(Number of particles adsorbed on the whole cuticle)}} \times 100$$

A: At least 70%; and
C: Lower than 70%.

(2) Evaluation of amount of particles adsorbed:

The number of particles adsorbed on the hair per surface area of 1,000 $\mu m^2$ was counted through an electron microscope to find its average value, thereby ranking the amount of particles adsorbed in accordance with the following standard.

A: At least 5 particles per 1,000 $\mu m^2$; and
C: Less than 5 particles per 1,000 $\mu m^2$.

(3) Evaluation of gloss or luster enhancing effect:

Ten panelists visually evaluated the gloss or luster enhancing effect as to each monitor, thereby ranking it in accordance with the following standard.

AA: All the ten panelists evaluated that the invention composition was effective as compared with the fine particle-free hair rinse composition (Comparative Product 1);
A: Seven to nine panelists evaluated that the invention composition was effective as compared, with the fine particle-free hair rinse composition (Comparative Product 1);
B: Four to six panelists evaluated that the invention composition was effective as compared with the fine particle-free hair rinse composition (Comparative Product 1); and
C: At most three panelists evaluated that the invention composition was effective as compared with the fine particle-free hair rinse composition (Comparative Product 1).

(4) Evaluation of effect of enhancing hair-dressing ability

Ten panelists visually evaluated the effect of enhancing hair-dressing ability as to each monitor, thereby ranking it in accordance with the following standard.

AA: All the ten panelists evaluated that the invention composition was effective as compared with the fine particle-free hair rinse composition (Comparative Product 1);
A: Seven to nine panelists evaluated that the invention composition was effective as compared with the fine particle-free hair rinse composition (Comparative Product 1);
B: Four to six panelists evaluated that the invention composition was effective as compared with the fine particle-free hair rinse composition (Comparative Product 1); and
C: At most three panelists evaluated that the invention composition was effective as compared with the fine particle-free hair rinse composition (Comparative Product 1).

Table 1

| Component (wt.%) | Comp. product | | |
|---|---|---|---|
| | 1 | 2 | 3 |
| Hydroxyethyl cellulose | 1 | 1 | 1 |
| Polyoxypropylene (30) polyoxyethylene (4) lauryl ether phosphate | 1 | 1 | 1 |
| Polyoxyethylene (5) oleyl ether | 3 | 3 | 3 |
| Polyoxyethylene (40) hardened castor oil | 0.5 | 0.5 | 0.5 |
| Stearyltrimethylammonium chloride | 1 | 1 | 1 |
| Liquid paraffin | 0.5 | 0.5 | 0.5 |
| Propylene glycol | 15 | 15 | 15 |
| Ethanol | 5 | 5 | 5 |
| Sodium hydroxide | 0.1 | 0.1 | 0.1 |
| Fine particles of titanium oxide (spherical, average particle diameter: 0.02 μm)*1 | - | 1 | - |
| Fine particles of silicone resin (spherical, average particle diameter: 0.5 μm)*2 | - | - | - |
| Fine particles of silicone resin (spherical, average particle diameter: 4.5 μm)*3 | - | - | 1 |
| Fine particles of polymethyl methacrylate (spherical, average particle diameter: 0.15 μm)*4 | - | - | - |
| Fine particles of polymethyl methacrylate (spherical, average particle diameter: 0.8 μm)*5 | - | - | - |
| Fine particles of polymethyl methacrylate (spherical, average particle diameter: 5.0 μm)*6 | - | - | - |
| Fine particles of styrene-divinylbenzene copolymer (flat, average particle diameter: 0.5 μm)*7 | - | - | - |
| Ion-exchanged water | Bal. | Bal. | Bal. |
| Selective adsorptivity on the edge part | - | A | A |
| Amount of particles adsorbed | - | A | C |
| Gloss or luster improving effect | - | B | B |
| Effect of enhancing hair-dressing ability | - | B | B |

*1: Idemitsu Titania IT-S (product of Idemitsu Kosan co., Ltd.).
*2: Tospearl® 105 (product of Toshiba Silicone Co., Ltd.).
*3: Tospearl® 145A (product of Toshiba Silicone Co., Ltd.).
*4: MP-1451 (product of Soken Chemical & Engineering Co., Ltd.).
*5: MP-1600 (product of Soken Chemical & Engineering Co., Ltd.).
*6: MX-500 (product of Soken Chemical & Engineering Co., Ltd.).
*7: MUTICLE® PP240D (product of Mitsui-Toatsu Chemicals, Inc.).

Table 2

| Component (wt.%) | Comp. product | |
|---|---|---|
| | 4 | 5 |
| Hydroxyethyl cellulose | 1 | 1 |
| Polyoxypropylene (30) polyoxyethylene (4) lauryl ether phosphate | 1 | 1 |
| Polyoxyethylene (5) oleyl ether | 3 | 3 |
| Polyoxyethylene (40) hardened castor oil | 0.5 | 0.5 |
| Stearyltrimethylammonium chloride | 1 | 1 |
| Liquid paraffin | 0.5 | 0.5 |
| Propylene glycol | 15 | 15 |
| Ethanol | 5 | 5 |
| Sodium hydroxide | 0.1 | 0.1 |
| Fine particles of titanium oxide (spherical, average particle diameter: 0.02 μm)*1 | - | - |
| Fine particles of silicone resin (spherical, average particle diameter: 0.5 μm)*2 | - | - |

*1: Idemitsu Titania IT-S (product of Idemitsu Kosan co., Ltd.).
*2: Tospearl® 105 (product of Toshiba Silicone Co., Ltd.).

Table 2 (continued)

| Component (wt.%) | Comp. product | |
|---|---|---|
| | 4 | 5 |
| Fine particles of silicone resin (spherical, average particle diameter: 4.5 μm)*3 | - | - |
| Fine particles of polymethyl methacrylate (spherical, average particle diameter: 0.15 μm)*4 | 1 | - |
| Fine particles of polymethyl methacrylate (spherical, average particle diameter: 0.8 μm)*5 | - | - |
| Fine particles of polymethyl methacrylate (spherical, average particle diameter: 5.0 μm)*6 | - | 1 |
| Fine particles of styrene-divinylbenzene copolymer (flat, average particle diameter: 0.5 μm)*7 | - | - |
| Ion-exchanged water | Bal. | Bal. |
| Selective adsorptivity on the edge part | A | A |
| Amount of particles adsorbed | A | C |
| Gloss or luster improving effect | B | B |
| Effect of enhancing hair-dressing ability | B | B |

*3: Tospearl® 145A (product of Toshiba Silicone Co., Ltd.).
*4: MP-1451 (product of Soken Chemical & Engineering Co., Ltd.).
*5: MP-1600 (product of Soken Chemical & Engineering Co., Ltd.).
*6: MX-500 (product of Soken Chemical & Engineering Co., Ltd.).
*7: MUTICLE® PP240D (product of Mitsui-Toatsu Chemicals, Inc.).

Table 3

| Component (wt.%) | Invention product | | | |
|---|---|---|---|---|
| | 1 | 2 | 3 | 4 4 |
| Hydroxyethyl cellulose | 1 | 1 | 1 | 1 |
| Polyoxypropylene (30) polyoxyethylene (4) lauryl ether phosphate | 1 | 1 | 1 | 1 |
| Polyoxyethylene (5) oleyl ether | 3 | 3 | 3 | 3 |
| Polyoxyethylene (40) hardened castor oil | 0.5 | 0.5 | 0.5 | 0.5 |
| Stearyltrimthylammonium chloride | 1 | 1 | 1 | 1 |
| Liquid paraffin | 0.5 | 0.5 | 0.5 | 0.5 |
| Propylene glycol | 15 | 15 | 15 | 15 |
| Ethanol | 5 | 5 | 5 | 5 |
| Sodium hydroxide | 0.1 | 0.1 | 0.1 | 0.1 |
| Fine particles of titanium oxide (spherical, average particle diameter: 0.02 μm)*1 | - | - | - | - |
| Fine particles of titanium oxide (spherical, average particle diameter: 0.6 μm)*8 | - | - | - | - |
| Fine particles of silicone resin (spherical, average particle diameter: 0.5 μm)*2 | 0.1 | 3 | - | 1 |
| Fine particles of silicone resin (spherical, average particle diameter: 4.5 μm)*3 | - | - | - | - |
| Fine particles of polymethyl methacrylate (spherical, average particle diameter: 0.15 μm)*4 | - | - | - | - |
| Fine particles of polymethyl methacrylate (spherical, average particle diameter: 0.8 μm)*5 | - | - | 1 | - |
| Fine particles of polymethyl methacrylate (spherical, average particle diameter: 5.0 μm)*6 | - | - | - | - |

*1: Idemitsu Titania IT-S (product of Idemitsu Kosan co., Ltd.).
*2: Tospearl® 105 (product of Toshiba Silicone Co., Ltd.).
*3: Tospearl® 145A (product of Toshiba Silicone Co., Ltd.).
*4: MP-1451 (product of Soken Chemical & Engineering Co., Ltd.).
*5: MP-1600 (product of Soken Chemical & Engineering Co., Ltd.).
*6: MX-500 (product of Soken Chemical & Engineering Co., Ltd.).
*8: Spherical $TiO_2$ particles (product of Hokko Chemical Industry co., Ltd.).

Table 3 (continued)

| Component (wt.%) | Invention product | | | |
|---|---|---|---|---|
| | 1 | 2 | 3 | 4 4 |
| Fine particles of styrene-divinylbenzene copolymer (flat, average particle diameter: 0.5 μm)*7 | - | - | - | 1 |
| Si-Black BL-100 (spherical, 0.25 μm)*9 | - | - | - | - |
| Ion-exchanged water | Bal. | Bal. | Bal. | Bal. |
| Selective adsorptivity on the edge part | A | A | A | A |
| Amount of particles adsorbed | A | A | A | A |
| Gloss or luster improving effect | AA | AA | AA | AA |
| Effect of enhancing hair-dressing ability | A | AA | AA | AA |

*7: MUTICLE® PP240D (product of Mitsui-Toatsu Chemicals, Inc.).
*9: Fine particles of surface-treated black iron oxide (product of Titan Kogyo K.K.).

Table 4

| Component (wt.%) | Invention product | | | |
|---|---|---|---|---|
| | 5 | 6 | 7 | 8 |
| Hydroxyethyl cellulose | 1 | 1 | 1 | 1 |
| Polyoxypropylene (30) polyoxyethylene (4) lauryl ether phosphate | 1 | 1 | 1 | 1 |
| Polyoxyethylene (5) oleyl ether | 3 | 3 | 3 | 3 |
| Polyoxyethylene (40) hardened castor oil | 0.5 | 0.5 | 0.5 | 0.5 |
| Stearyltrimethylammonium chloride | 1 | 1 | 1 | 1 |
| Liquid paraffin | 0.5 | 0.5 | 0.5 | 0.5 |
| Propylene glycol | 15 | 15 | 15 | 15 |
| Ethanol | 5 | 5 | 5 | 5 |
| Sodium hydroxide | 0.1 | 0.1 | 0.1 | 0.1 |
| Fine particles of titanium oxide (spherical, average particle diameter: 0.02 μm)*1 | - | - | - | - |
| Fine particles of titanium oxide (spherical, average particle diameter: 0.6 μm)*8 | 1 | - | - | - |
| Fine particles of silicone resin (spherical, average particle diameter: 0.5 μm)*2 | - | - | - | - |
| Fine particles of silicone resin (spherical, average particle diameter: 4.5 μm)*3 | - | - | - | - |
| Fine particles of polymethyl methacrylate (spherical, average particle diameter: 0.15 μm)*4 | - | - | - | - |
| Fine particles of polymethyl methacrylate (spherical, average particle diameter: 0.8 μm)*5 | - | - | - | - |
| Fine particles of polymethyl methacrylate (spherical, average particle diameter: 5.0 μm)*6 | - | - | - | - |
| Fine particles of styrene-divinylbenzene copolymer (flat, average particle diameter: 0.5 μm)*7 | - | - | - | - |
| Si-Black BL-100 (spherical, 0.25 μm)*9 | - | 1 | - | - |

*1: Idemitsu Titania IT-S (product of Idemitsu Kosan co., Ltd.).
*2: Tospearl® 105 (product of Toshiba Silicone Co., Ltd.).
*3: Tospearl® 145A (product of Toshiba Silicone Co., Ltd.).
*4: MP-1451 (product of Soken Chemical & Engineering Co., Ltd.).
*5: MP-1600 (product of Soken Chemical & Engineering Co., Ltd.).
*6: MX-500 (product of Soken Chemical & Engineering Co., Ltd.).
*7: MUTICLE® PP240D (product of Mitsui-Toatsu Chemicals, Inc.).
*8: Spherical $TiO_2$ particles (product of Hokko Chemical Industry co., Ltd.).
*9: Fine particles of surface-treated black iron oxide (product of Titan Kogyo K.K.).

Table 4 (continued)

| Component (wt.%) | Invention product | | | |
|---|---|---|---|---|
| | 5 | 6 | 7 | 8 |
| Fine particles of chitosan-methacrylic acid copolymer (spherical, average particle diameter: 0.9 μm) | - | - | 2 | - |
| Fine particles of styrene-divinylbenzene copolymer (spherical, average particle diameter: 0.5 μm)*10 | - | - | - | 1 |
| Ion-exchanged water | Bal. | Bal. | Bal. | Bal. |
| Selective adsorptivity on the edge part | A | A | A | A |
| Amount of particles adsorbed | A | A | A | A |
| Gloss or luster improving effect | AA | AA | AA | A |
| Effect of enhancing hair-dressing ability | AA | AA | AA | A |

*10: About 70% of the particles are within a range of the average particle diameter ± 30%, and about 30% of the particles are outside a range of the average particle diameter ± 30%.

Example 2:

Hair cosmetic compositions having their corresponding formulations shown in Table 5 were prepared in accordance with a method known *per se* in the art and separately sprayed on a bundle of hair. After the bundles of hair thus treated were air-dried, the hair cosmetic compositions were evaluated as to the selective adsorptivity on the edge part in the same manner as in Example 1. Besides, their gloss or luster enhancing effects were visually evaluated by 10 panelists. The results are shown in Table 5.

Table 5

| Component (wt.%) | Inv. Prod. 9 | Inv. Prod. 10 | Comp. Prod. 6 | Comp. Prod. 7 |
|---|---|---|---|---|
| Spherical fine particles of polymethyl methacylrate (0.8 μm)*5 | 0.05 | 0.05 | 0.05 | 0.05 |
| Stearyltrimethylammonium chloride | 0.03 | - | 0.00 08 | - |
| Polyglycol-polyamine condensed resin*11 | - | 0.05 | - | - |
| Coloring matter | q.s. | q.s. | | q.s. |
| Perfume base | q.s. | q.s. | q.s. | q.s. |
| Ion-exchanged water | Bal. | Bal. | q.s. Bal. | Bal. |
| Selective adsorptivity on the edge part | A | A | C | C |

*5: MP-1600 (product of Soken Chemical & Engineering co., Ltd.).

*11: Polyquat® NH (product of Henkel Hakusui Corporation).

Nine of 10 panelists evaluated that the bundles of hair sprayed with Invention Product 9 and Invention Product 10 are superior in both intensity of and preference in gloss to both Comparative Product 6 and Comparative Product 7.

Example 3:

A rinse composition was prepared in accordance with the following formulation.

| | | (wt.%) |
|---|---|---|
| (1) | Hydroxyethyl cellulose | 1 |
| (2) | Polyoxypropylene polyoxyethylene alkyl ether phosphate | 1 |
| (3) | Polyoxyethylene alkyl ether | 3 |
| (4) | Polyoxyethylene hardened castor oil | 0.5 |
| (5) | Stearyltrimethylammonium chloride | 1 |
| (6) | Liquid paraffin | 0.5 |

(continued)

| | | (wt.%) |
|---|---|---|
| (7) | Propylene glycol | 15 |
| (8) | Ethanol | 5 |
| (9) | Sodium hydroxide | 0.1 |
| (10) | Ion-exchanged water | 65.9 |
| (11) | Dispersion of canonized fine particle of polymethyl methacrylate *12 | 7.0 |

*12: Obtained by adding 10 g of fine particles*13 of polymethyl methacrylate to 60 g of a 0.2% aqueous solution of stearyltrimethylammonium chloride and stirring the mixture into a dispersion.
*13: Fine particles of polymethyl methacrylate, MP-5000 (spherical; average particle diameter: 0.4 μm; product of Soken Chemical & Engineering Co., Ltd.).

After components (1), (3) to (7) and (10) were heated to 70°C and mixed, the mixture was cooled down to 50°C, and component (2), (8), (9) and (11) were added thereto. The resultant mixture was cooled with stirring. Examples 4 and 5:

Rinse compositions were prepared in accordance with the following respective formulations.

| | | (wt.%) | |
|---|---|---|---|
| | | Ex. 4 | Ex. 5 |
| (1) | Hydroxyethyl cellulose | 1 | 1 |
| (2) | Polyoxypropylene polyoxyethylene alkyl ether phosphate | 1 | 1 |
| (3) | Polyoxyethylene alkyl ether | 3 | 3 |
| (4) | Polyoxyethylene hardened castor oil | 0.5 | 0.5 |
| (5) | Stearyltrimethylammonium chloride | 1 | 1 |
| (6) | Liquid paraffin | 0.5 | 0.5 |
| (7) | Propylene glycol | 15 | 15 |
| (8) | Ethanol | 5 | 5 |
| (9) | Sodium hydroxide | 0.1 | 0.1 |
| (10) | Fine particles of styrene-methacrylic acid copolymer*14 | 1.0 | - |
| (11) | Fine particles of polymethyl methacrylate*15 | - | 1.0 |
| (12) | Ion-exchanged water | Bal. | Bal. |

*14: Positively charged particles, MP-5500 (+450 μc/g; spherical; average particle diameter: 0.4 μm; product of Soken Chemical & Engineering Co., Ltd.).

*15: Positively charged particles, MP-2701 (+500 μc/g; spherical; average particle diameter: 0.4 μm; product of Soken Chemical & Engineering Co., Ltd.).

Example 6:

A hair spray composition was prepared by mixing the following components.

| | | (wt.%) |
|---|---|---|
| (1) | Dispersion of canonized styrene-divinylbenzene-alkyl acrylate terpolymer*16 | 1.5 |
| (2) | Ethanol | 5.0 |
| (3) | Perfume base | q.s. |
| (4) | Coloring matter | q.s. |
| (5) | Ion-exchanged water | Balance |

*16: Obtained by adding 0.16 g of styrene-divinylbenzene-acrylic acid terpolymer*17 to 50 g of a 0.16% aqueous solution of stearyl-trimethylammonium chloride and stirring the mixture into a dispersion.
*17: MUTICLE® PP110C (indeterminate form; average particle diameter: 1.0 μm; product of Mitsui-Toatsu Chemicals, Inc.).

Example 7:

A hair spray composition was prepared by mixing the following components.

| | | (wt.%) |
|---|---|---|
| (1) | Aqueous dispersion of canonized $TiO_2$ | 25 |
| (2) | Perfume base | q.s. |
| (3) | Coloring matter | q.s. |
| (4) | Aminoethylaminopropylsiloxane-dimethylsiloxane copolymer emulsion*19 | 0.25 |
| (5) | Ion-exchanged water | Balance |

*18: Obtained by adding 0.02 g of $TiO_2$*20 to 50 g of a 0.3% aqueous solution of Celquat® L-200*19 and stirring the mixture into a dispersion.
*19: Product of National Starch Co.;
polyquaternium-4.
*20: Spherical $TiO_2$ particles (average particle diameter: 0.6 μm; product of Hokko Chemical Industry Co., Ltd.).
*21: SM8702C (product of Dow Corning Toray Silicone Co., Ltd.).

Example 8:

A hair spray composition was prepared by mixing the following components.

| | | (wt.%) |
|---|---|---|
| (1) | Aqueous dispersion of canonized $TiO_2$*22 | 25 |
| (2) | Perfume base | q.s. |
| (3) | Coloring matter | q.s. |
| (4) | Diethylene glycol monoethyl ether | 20 |
| (5) | Ion-exchanged water | Balance |

*22: Obtained by adding 0.12 g of $TiO_2$*20 to 100 g of a 3% aqueous solution of Gafquat® 734*23 and stirring the mixture into a dispersion.
*23: Product of ISP Co.; polyquaternium-11.

Comparative Example 8:

A rinse composition was prepared in the same manner as in Example 3 except that ion-exchanged water was supplemented in place of the component (11).

Comparative Example 9:

A hair spray composition was prepared in the same manner as in Example 6 except that ion-exchanged water was supplemented in place of the component (1).

Comparative Example 10:

A hair spray composition was prepared in the same manner as in Example 7 except that a 0.3% aqueous solution of Celquat® L-200 was used in place of the component (1).

Comparative Example 11:

A hair spray composition was prepared in the same manner as in Example 8 except that a 3% aqueous solution of Gafquat® 734 was used in place of the component (1).

Test Example:

Women monitors separately used the hair cosmetic compositions obtained in Examples 3-8 and Comparative Examples 8-11, and ten panelists visually evaluated the respective compositions as to the gloss or luster enhancing effect and the effect of enhancing hairdressing ability.
The evaluation was conducted by comparison between the compositions of Examples 3 to 5 and the composition of Comparative Example 8, between the composition of Example 6 and the composition of Comparative Example 9, between the composition of Example 7 and the composition of Comparative Example 10, and between the composition of Example 8 and the composition of Comparative Example 11, thereby ranking them in accordance with the following standard.

AA: All the ten panelists evaluated that the composition of Example was more effective than the comparative composition;

A: Seven to nine panelists evaluated that the example composition was more effective than the comparative composition;

B: Four to six panelists evaluated that the example composition was more effective than the comparative composition; and

C: At most three panelists evaluated that the example composition was more effective than the comparative composition.

Table 6

| | Examples 3, 4, 5 | Example 6 | Example 7 | Example 8 |
|---|---|---|---|---|
| Gloss or luster enhancing effect | A A A | AA | A | A |
| Effect of improving hair-dressing ability | A A A | A | A | A |

According to the hair cosmetic compositions of the present invention, the water-insoluble fine particles incorporated therein are selectively adsorbed on the edge part of the hair cuticle, thereby permitting imparting natural gloss or luster to hair, giving users a pleasant feel without any sticky feel and enhancing hair-dressing ability.

This invention has been disclosed in terms of generic description and by specific example.

### Claims

1. A hair cosmetic composition comprising fine particles of a water-insoluble polymer or inorganic material, which have an average particle diameter not smaller than 0.2 µm, but smaller than 1 µm.

2. The hair cosmetic composition according to Claim 1, wherein the average particle diameter of the fine particles is 0.3 to 0.9 ii-m.

3. The hair cosmetic composition according to Claim 1, wherein the particle size distribution of the fine particles is such that at least 80% of the fine particles are within a range of the average particle diameter ± 30%.

4. The hair cosmetic composition according to Claim 1, wherein the fine particles selectively adsorb on an edge part of hair cuticle.

5. The hair cosmetic composition according to Claim 1, wherein the fine particles are those of a water-insoluble polymer selected from the group consisting of silicone resins, polymers of alkyl (meth)acrylates and copolymers thereof with monomers copolymerizable therewith, polymers of styrene and copolymers thereof with monomers copolymerizable therewith, and chitosan-methacrylic acid copolymers, or fine particles of an inorganic material selected from the group consisting of titanium oxide, silica, alumina, iron oxide, zinc oxide, chromium oxide and mica.

6. The hair cosmetic composition according to Claim 1, wherein the fine particles are those of a water-insoluble, cationic polymer.

7. The hair cosmetic composition according to Claim 1, wherein the fine particles are fine particles surface-treated with a cationic compound.

8. The hair cosmetic composition according to Claim 1, which further comprises a cationic compound.

9. The hair cosmetic composition according to Claim 1, wherein the fine particles are contained in an amount of 0.001-10 wt.%.

10. The hair cosmetic composition according to Claim 1, wherein the fine particles are contained in an amount of 0.001-0.1 wt.%, and the composition is one which is not rinsed off with water after application to hair.

**11.** The hair cosmetic composition according to Claim 1, wherein the fine particles are contained in an amount of 0.01-10 wt.%, and the composition is one which is rinsed off with water after application to hair.

**12.** A method of imparting gloss or luster to hair, which comprises applying the hair cosmetic composition according to Claim 1 to the hair.

**13.** A method of imparting gloss or luster to hair, which comprises applying fine particles of a water-insoluble polymer or inorganic material, which have an average particle diameter not smaller than 0.2 μm, but smaller than 1 μm, to the hair.

**Patentansprüche**

**1.** Haarkosmetische Zusammensetzung, umfassend feine Teilchen eines wasserunlöslichen Polymers oder anorganischen Materials, die einen durchschnittlichen Teilchendurchmesser von nicht kleiner als 0,2 μm, aber kleiner als 1 μm aufweisen.

**2.** Haarkosmetische Zusammensetzung nach Anspruch 1, wobei der durchschnittliche Teilchendurchmesser der feinen Teilchen 0,3 bis 0,9 μm beträgt.

**3.** Haarkosmetische Zusammensetzung nach Anspruch 1, wobei die Teilchengrössenverteilung der feinen Teilchen derart ist, dass mindestens 80 % der feinen Teilchen in einem Bereich des durchschnittlichen Teilchendurchmessers von ± 30 % sind.

**4.** Haarkosmetische Zusammensetzung nach Anspruch 1, wobei die feinen Teilchen selektiv am Randteil des Haaroberhäutchens adsorbieren.

**5.** Haarkosmetische Zusammensetzung nach Anspruch 1, wobei die feinen Teilchen solche eines wasserunlöslichen Polymers sind, ausgewählt aus der Gruppe bestehend aus Siliconharzen, Polymeren von Alkyl(meth)acrylaten und Copolymeren hiervon mit hiermit copolymerisierbaren Monomeren, Polymeren von Styrol und Copolymeren hiervon mit hiermit copolymerisierbaren Monomeren, und Chitosan-Methacrylsäure-Copolymeren, oder feine Teilchen eines anorganischen Materials, ausgewählt aus der Gruppe bestehend aus Titanoxid, Siliciumoxid, Aluminiumoxid, Eisenoxid, Zinkoxid, Chromoxid und Glimmer.

**6.** Haarkosmetische Zusammensetzung nach Anspruch 1, wobei die feinen Teilchen solche eines wasserunlöslichen kationischen Polymers sind.

**7.** Haarkosmetische Zusammensetzung nach Anspruch 1, wobei die feinen Teilchen mit einer kationischen Verbindung oberflächenbehandelte feine Teilchen sind.

**8.** Haarkosmetische Zusammensetzung nach Anspruch 1, weiterhin umfassend eine kationische Verbindung.

**9.** Haarkosmetische Zusammensetzung nach Anspruch 1, in der die feinen Teilchen in einer Menge von 0,001 bis 10 Gew.% enthalten sind.

**10.** Haarkosmetische Zusammensetzung nach Anspruch 1, in der die feinen Teilchen in einer Menge von 0,001 bis 0,1 Gew.% enthalten sind und die Zusammensetzung eine solche ist, die nach dem Auftragen auf das Haar nicht mit Wasser abgespült wird.

**11.** Haarkosmetische Zusammensetzung nach Anspruch 1, in der die feinen Teilchen in einer Menge von 0,01 bis 10 Gew.% enthalten sind, und die Zusammensetzung eine solche ist, die nach dem Auftragen auf das Haar mit Wasser abgespült wird.

**12.** Verfahren, um das Haar glänzend und schimmernd zu machen, umfassend das Auftragen der haarkosmetischen Zusammensetzung gemäss Anspruch 1 auf das Haar.

**13.** Verfahren, um das Haar glänzend und schimmernd zu machen, umfassend das Auftragen feiner Teilchen eines wasserunlöslichen Polymers oder eines anorganischen Materials, die einen durchschnittlichen Teilchendurchmesser von nicht kleiner als 0,2 µm, aber kleiner als 1 µm, aufweisen, auf das Haar.

**Revendications**

1. Composition pour soins capillaires, comprenant de fines particules d'un polymère insoluble dans l'eau ou de matières inorganiques qui ont un diamètre particulaire moyen non inférieur à 0,2 µm, mais inférieur à 1 µm.

2. Composition pour soins capillaires selon la revendication 1, dans laquelle le diamètre particulaire moyen des particules fines est de 0,3 à 0,9 µm.

3. Composition pour soins capillaires selon la revendication 1, dans laquelle la répartition des tailles particulaires des particules fines est telle qu'au moins 80% des particules fines se situent dans une plage du diamètre particulaire moyen de plus ou moins 30%.

4. Composition pour soins capillaires selon la revendication 1, dans laquelle les particules fines adsorbent sélectivement sur une partie marginale de cuticule capillaire.

5. Composition pour soins capillaires selon la revendication 1, dans laquelle les particules fines sont celles d'un polymère insoluble dans l'eau, choisi dans le groupe consistant en résines de silicone, polymères d'alkyle (méth) acrylates et leurs copolymères avec des monomères copolymérisables avec ceux-ci, des polymères de styrène et leurs copolymères avec des monomères copolymérisables avec ceux-ci, et des copolymères de l'acide chitosan-méthacrylique, ou des particules fines d'un matériau inorganique choisi dans le groupe consistant en oxyde de titane, silice, alumine, oxyde de fer, oxyde de zinc, oxyde de chrome et mica.

6. Composition pour soins capillaires selon la revendication 1, dans laquelle les particules fines sont celles d'un polymère cationique insoluble dans l'eau.

7. Composition pour soins capillaires selon la revendication 1, dans laquelle les particules fines sont des particules fines dont la surface est traitée avec un composé cationique.

8. Composition pour soins capillaires selon la revendication 1, comprenant de plus un composé cationique.

9. Composition pour soins capillaires selon la revendication 1, dans laquelle les particules fines sont contenues dans une quantité de 0,001-10% en poids.

10. Composition pour soins capillaires selon la revendication 1, dans laquelle les particules sont contenues dans une quantité de 0,001-0,1% en poids et la composition est une composition qui n'est pas éliminée par rinçage à l'eau après l'application sur les cheveux.

11. Composition pour soins capillaires selon la revendication 1, dans laquelle les particules fines sont contenues dans une quantité de 0,01-10% en poids, et la composition est une composition qui est éliminée par rinçage à l'eau après l'application sur les cheveux.

12. Procédé destiné à conférer du soyeux ou de l'éclat aux cheveux, comprenant l'application sur les cheveux de la composition pour soins capillaires selon la revendication 1.

13. Procédé destiné à conférer du soyeux ou de la brillance aux cheveux, comprenant l'application sur les cheveux de fines particules d'un polymère insoluble dans l'eau ou d'une matière inorganique, qui ont un diamètre particulaire moyen non inférieur à 0,2 µm, mais inférieur à 1 µm.

Fig. 1 (Invention product 9)

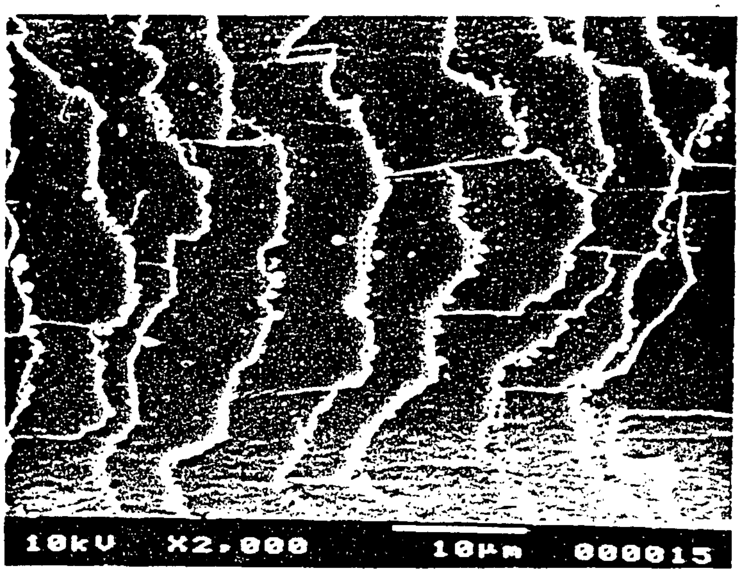


Fig. 2 (Invention product 10)

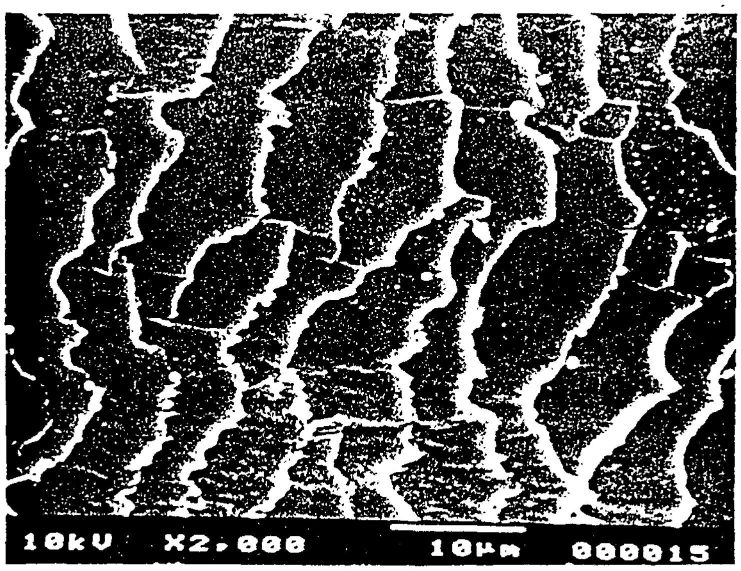

Fig. 3 (Comparative product 6)

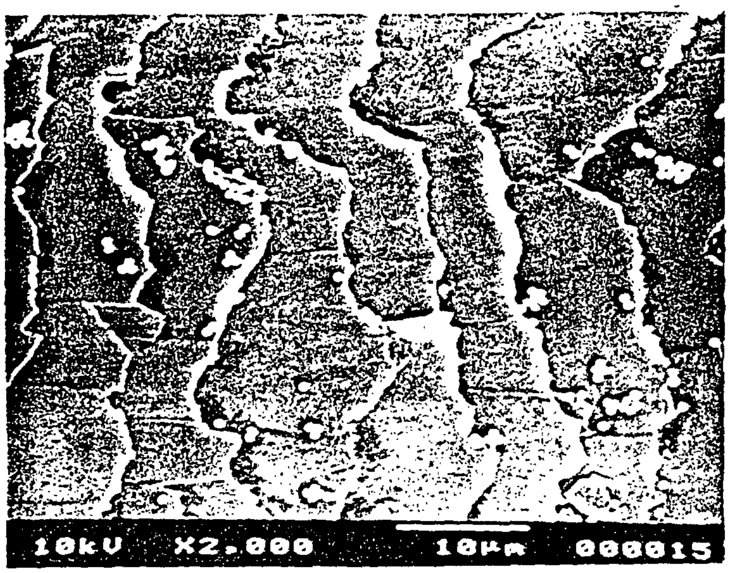


Fig. 4 (Comparative product 7)